# EUROPEAN PATENT APPLICATION

(11) **EP 3 760 211 A1**
(43) Date of publication of application: **06.01.2021**
(21) Application number: 20000234.3
(22) Date of filing: 30.06.2020
(51) Int. Cl.: A61K 35/618, A61P 17/02

(54) **LYOPHILIZED SNAIL DROOL, USE OF SUCH DROOL AND PRODUCTS OBTAINED THEREFROM**

(30) Priority: 03.07.2019 IT 201900010827
(71) Applicant: C.T.S.V. S.R.L., 25124 Brescia (IT); Romussi, Davide, 10050 Chiomonte (TO) (IT); Bodoira, Sara, 10075 Mathi (TO) (IT); Icardi, Alessandro, 14014 Capriglio (AT) (IT)
(72) Inventor: Romussi, Davide, I-10050 Chiomonte (TO) (IT); Icardi, Alessandro, I-14014 Capriglio (AT) (IT)
(74) Representative: Garavelli, Paolo

(57) **Abstract**

Lyophilized snail drool to be used as cicatrizer and/or as regenerative surgical support, the use of the lyophilized snail drool as lyophilized cicatrizer or as regenerative surgical support, a regenerative surgical support obtained from compacting the lyophilized snail drool, a disposable active patch comprising lyophilized snail drool are described; a process for producing lyophilized snail drool is further described.

## Description

The present invention refers to lyophilized snail drool, to the use of such lyophilized snail drool as cicatrizer, to the use with regenerative surgical supports, with a scaffold for a surgical bio-scaffold, or with a disposable active patch; in particular, the invention refers to a snail drool or "snail slime", known as snail secretion filtrate in the INCI (International Nomenclature of Cosmetic Ingredients) denomination, preferably produced by snails of the Helix Aspersa Muller species.

The use of snail drool ("snail slime", snail secretion filtrate in the INCI (International Nomenclature of Cosmetic Ingredients) denomination) is known in the medical and cosmetic fields for its decongestant and anti-inflammatory properties to soothe skin irritations and inflammations, since the Middle Ages, wherein the snail drool had been widely employed in the field of traditional medicine, to cure stomach problems, such as gastritis and peptic ulcers, cicatrize the wounds and stop hemorrhages, and moreover, as syrup, dissolve the phlegm and enable its removal, in addition to calm a cough.

More recently, the snail drool has become an important component of many creams and ointments, which have the power of making a skin more tonic and hydrated, giving it a fresh and stretched appearance, following the discovery, occurred in snail farms for food purposes, that the operators working in contact with them had an extraordinary soft and velvety skin. This revelation ended up in being highly interesting by researchers and scholars, who started analyzing the properties of the drool produced by the Helix Aspersa Muller species, making it become an important component of many creams and ointments.

Synthetically, the following properties of the snail drool are known:
- Moisturizing: due to muco-polysaccharides, film-forming substances which have the power of keeping water on the skin and ensure a long-lasting hydrating effect;
- Cicatrizing: due to the combined effect of some substances, such as collagen, allantoin and vitamins;

- Soothing: useful for reddening due to the prolonged exposure to sun or cold;
- Regenerating and nutritive: due to the strong presence of vitamins and proteins;
- Wrinkle-preventing: it stimulates the synthesis of the collagen, due to the action of the glycolic acid combined with elastin, muco-polysaccharides, vitamins and collagen;
- Stretch marks-preventing: for the regenerating action of vitamins and proteins and the exfoliating action of the glycolic acid;
- Efficient against blemishes and signs of acne: due to the smoothing, exfoliating and purifying properties of the glycolic acid, further powered by the soothing effect of the other components.

The main known components of snail drool are the following:
- Allantoin: several studies demonstrate that the allantoin contained in the snail drool, also known with the chemical name glyoxyl di-urea, stimulates the epithelialization (epithelium reconstruction) of the skin, stimulating the cellular proliferation. The allantoin further helps removing the dead cells of the necrotic tissue and acts on the regeneration of new cells.

Moreover, the allantoin has been studied to have a protective effect on the skin, preventing chemical substances contained in some soaps and oils for caring the skin which abuse it. The allantoin is a natural irritation-preventing substance. In particular, the FDA (Food and Drug Administration) and the EMA (European Medicines Agency) approve the use of allantoin in skin creams, as useful ingredient in the cosmetic field.
- Glycolic acid: the glycolic acid exfoliates and allows peeling (desquamation) of the epidermis, removing dead cells which are found on the surface of the skin. Moreover, even more important, the glycolic acid gives a contribution to cleaning the epidermis, enabling the penetration of the other components present in the snail drool cream through the hair follicles to exert their therapeutic properties on the deepest layers of the skin.
- Proteins and Vitamins: the snail drool contains several vitamins (in particular vitamin C and vitamin E) and proteins which an animal produces through its vegetable diet. Proteins help feeding the skin and making it softer. Vitamins enforce the action of natural antibiotics, also enabling to heal possible injuries.
- Natural antibiotics: the natural antibiotics present in the snail drool are substances capable of fighting the infections caused da bacteria usually present on the skin, such as *Staphylococcus aureus, Escherichia coli, Pseudomonas aeruginosa,* and the whole family of bacteria involved in acne. It is also for this reason that it is very efficient in depurating the skin affected by bacterial impurities which often cause acne.
- Collagen and Elastin: these two important ingredients of the connective tissue of the skin can be naturally and abundantly found in the extract of snail drool. Collagen is an essential part of the dermis which gives the skin softness and compactness, above all when it is associated with elastin.

Elastin prevents the skin ageing. As the term suggests, elastin allows the tissues to elongate and go back to their initial status after stretching, conferring flexibility and elasticity to the epidermis.

As regards husbandry, the term "elicicolture" identifies the natural breeding performed both inside and outside. If, at the beginning, the purpose of this practice was typically food, the breeders afterwards focused on the extraction of the drool and the sale of this substance to companies active in the cosmetic field. Nowadays, cosmetic firms have finally abolished the use of drool coming from farms which to not respect the snail life, pushing towards a production where these clams are respected and preserved.

In the majority of cases, current farms are made in the open, using manual and not any more electric stimulations, as happened in the past. Upon extracting the drool, snails are collected, cleaned from residuals of ground and placed inside particular tubs, inside which this precious substance is collected. Inside these vessels, animals are delicately moved, in order to stimulate the secretion of drool. Once having filled in the vessel bottom, snails are moved, washed and placed again in the farm. The process is cyclic and is carried out using always different clams, in order not to impair the animal integrity. No external stimulation is applied, which can imply damages or nuisance. Afterwards, the drool is micro-filtered and used for producing medicines and cosmetics.

Object of the present invention is providing lyophilized snail drool which allows obtaining, in a completely natural way, free from additives and/or manipulations, an innovative bio-material having concentrations which cannot be found in nature apart from synthesis products, to use the obtained product as co-adjuvant of dermal and epidermal lesions, on the surface and deep, young or chronic of a physiological or mechanical nature.

Other objects deal with the use of such lyophilized snail drool as cicatrizer, with a scaffold for surgical bio-scaffold, or with a disposable active patch, and the making of products for such uses.

The above and other objects and advantages of the invention, as will result from the following description, are obtained with the lyophilized snail drool, and with the use of such lyophilized snail drool as cicatrizer, with a scaffold for surgical bio-scaffold, or with a disposable active patch, as claimed in the independent claims, Preferred embodiments and non-trivial variations of the present invention are the subject matter of the dependent claims.

It is intended that all enclosed claims are an integral part of the present description.

It will be immediately obvious that numerous variations and modifications (for example related to shape, sizes, arrangements and parts with equivalent functionality) can be made to what is described, without departing from the scope of the invention as included in the enclosed claims.

The present invention will be better described by some preferred embodiments thereof, provided as a non-limiting example.

As a non-limiting example a process is described below for extracting snail drool, for example from snails of the Helix Aspersa Muller species.

In order to produce a high-quality drool, the animal must live under welfare conditions; therefore, animals are bred only in an open field, with vegetables which are uses both for feeding and as guard against predators and against adverse weather conditions, such as excessive sun radiations and wind, which can bring about a quick dehydration of the snail body.

Since the drool quality depends on feeding and arrangement of the gastropods, gastropods are fed with specific vegetables like: kale, knight cabbage, proteor cabbage and cutting chard. Moreover, as exclusive protection, they are seeded together with the food vegetable small white clover. Another major condition for the animal welfare is keeping a high degree of humidity both aerial, namely in between the vegetation, and in the ground; fields are therefore irrigated daily, excluding particularly rainy days.

In order to limit escapes of snails from the field where they have been inserted and avoid the entry of predators, such as for example some types of insects, mice or hedgehogs, the ground is divided with fencing structures made of suitable materials and sizes and surrounded by a special perimeter fence. Corridors are created between the fences, for the passage of personnel responsible for the various activities of the plant. Vegetables are seeded exclusively inside the fences, while the passage corridors and the internal and external perimeter are free from weeds, in order to avoid that the snails stops there and eat them.

In order to proceed with a quick collection of the snails, which are subjected to the drool extraction, the farmer places wooden footboards inside the fences; snails love wood and love, during sun hours, to be protected under footboards, strongly enabling and accelerating the collection work.

Snails subjected to the collection treatment are adult, healthy from the point of view of their body mass, of the water content and alert. Upon collecting the snails from the footboards, the operator performs a first selection by choosing only the adults, which can be recognized by their thick and hard edges present in the shell mouth. Snails pre-selected in this way are taken in the so-called dirty area, which can be distinguished in a closed room, where they are weighed, further selected an washed.

Afterwards, the washed snails are taken in the clean area, the laboratory, where they are placed inside a drool-collecting machine. The machine takes care of collecting and filtering the drool, which is placed in suitable sterilized containers.

As a non-limiting example, a process is described for extracting the snail drool, which comprises the following steps:
- a first step, wherein the process starts with the introduction of an amount of snails equal to about 8/10 Kg, according to the size of the gastropod, contained in a static collection holed basket, inside a first bell, in order to guarantee enough life space to the snails; in the bell, the snails are subjected to washing through nebulization of ozonized water and treated with UV rays, for a preferred time of about 15 minutes. Observations performed during this step detected an optimum keeping of the animal welfare, demonstrated by the natural movement of the gastropods;

- a second step, wherein, after having been washed in the first step, snails are transferred by moving the collection basket from the first to a second bell, where stimulation will happen. The snails placed on the covering glass are collected manually one by one and placed in the basket;
- a third step, wherein the drool is extracted from the snails, preferably comprising two cycles with a length equal to 14 minutes each, for a total of 28 minutes. Every cycle comprises two nebulizing of a stimulating agent, containing Potassium Sorbate and depurated Water, and two nebulizing of water. As soon as the stimulating agent is nebulized, animals immediately start the drool collection without signals of suffering. At the end of the second cycle, the gastropods are washed with ozonized water.

Advantageously, the technology used in this process allows an animal not to suffer because the used principle is a stimulation to the secretion of the drool through nebulizing of an absolutely nontoxic natural compound.

The process is performed without mechanical stimulations such as shaking or manual scrubbing, thereby without the risk of damaging the snails;
- a fourth step, wherein, at the end of washing, the snails are accurately collected, placed in transparent containers and transported outside the laboratory for the final washing. After the collection of drool, the animals are alive and vital;
- a fifth step, wherein an accurate manual washing is performed in tepid water, with a following deposition of the gastropods in a resting cage before they are taken back in an open field, inserted in a different row with respect to the starting one, to avoid that an extraction cycle is again performed on the same snails. For the welfare of the animal, before being re-used for the collection of drool, it is necessary to wait for at least two weeks.

Upon the immediate observation in the transport containers and as soon as they are inserted in a resting cage, the snails are always alive and vital and responding to stimuli. Upon a further observation after 40 minutes in a resting cage, the animals appear alive, vital and moving.

Herein below, the features of a sample of snail drool are listed; in particular, from laboratory analyses, the following values have been detected for the major excipients, namely the concentration of glycolic acid, of allantoin and of proteins, which are perfectly compatible with the gold standard analyses owned by the scientific community:
- Glycolic acid: mean value 13447 mg/kg
- Allantoin: mean value 819 mg/kg
- Total proteins: mean value 0,6 g/100g
- pH: 3.01
- Collagen: nn
- Bacterial charge: 0% (zero)
- Conductivity and Electric Resistivity: 3001 Ohm (sample placed at the temperature of 37°C) .
- SS Percentage: 6.7%
- Concentration of heavy metals; scarce/null presence of heavy metals dangerous for the health, such as Cadmium, Lead, Nickel and Mercury.

Finally, the lyophilized snail drool of the invention has been obtained, for example using the lyophilizing process described below.

The liquid product (snail drool) has been stored in a refrigerating cell at a temperature included between 0 °C and -4 °C till the freezing preparatory to lyophilizing.

For example, from 1 liter of drool, 0.5 liters of product have been taken and is dispensed in eight small silicone tanks with a liquid thickness of 3 - 5 mm.

The above tanks have then been placed under freezing at -35 °C for about 24 hours. After the freezing operation has ended, the containers have been quickly placed on shelves of a lyophilizing tower and, after having completed the preparation of the plant, the lyophilizing cycle has started.

The lyophilizing parameters of the lyophilized snail drool of the invention are the following:
- temperature of about -50° C
- vacuum of about 0,020 mmbar

After 24 h, the lyophilized product is packaged.

For example, for lyophilizing a known lyophilized product has been used, of the Chris-Alpha type, following the procedure and the method of use included in the related instruction manual.

The tanks containing the liquid solution are made of a material complying with food use. The containers for storing the lyophilized end product are Kartell jars certified for a pharmaceutical use.

The lyophilized snail drool of the invention has a white color with shading tending to a cream color. The material is shaped as a crystal, very sticky and with strong hygroscopic features.

Laboratory analyses performed on the material obtained from lyophilizing (lyophilized snail drool) have provided the following results, which are surprisingly different from expected values as result of the lyophilizing process, reaching known values only for synthesis products or products of a vegetable origin.

The lyophilized product expresses values which can be clinically referred to applications which enable and help the skin proliferation, namely the care of generic and chronic injuries:
- Glycolic acid: mean value 227464 mg/kg, namely +5535% with respect to its starting raw material
- Allantoin: mean value 46147 mg/kg, namely +1592% with respect to its starting raw material
- Total proteins: mean value 2.1 g/100g, namely +250% with respect to their starting raw material
- pH: 3.5, namely +16.3% with respect to its starting raw material
- Collagen: 1% m/m
- Bacterial charge: 0% (zero), confirmed data.
- Concentration of heavy metals: scarce/null presence of heavy metals dangerous for the health, such as Cadmium, Lead, Nickel and Mercury, confirmed data.

The snail drool obtained after the lyophilizing process comprises the following percentages of components:
- Glycolic acid: included between 15% and 35%, preferably about 25%
- Allantoin: included between 2% and 8%, preferably about 5%
- Proteins: included between 2% and 6%, preferably about 4%
- Collagen: included between 0.5% and 1.5%, preferably about 1%
- Remaining: included between 50% and 80%, preferably 65% of a matrix with very low molecular weight which makes it very hygroscopic.

Surprisingly, the snail drool obtained after the lyophilizing process has unexpected values of glycolic acid and allantoin, in concentrations much higher than those expected as result of the lyophilizing process.

The lyophilized product expresses values which can be clinically referred to applications which enable and help the ski proliferation, namely the care of generic and chronic injuries.

The glycolic acid is a carboxylic acid own of the vegetable world, is a fruit acid which can be found, for example, in sugar cane, in beets and in unripe grapes.

The concentration of 25% of glycolic acid can be referred, nowadays, only to synthesis products and obviously in products of a vegetable origin, while it is not known for snail drool.

The lyophilized snail drool of the invention can be used in products as regenerative surgical support for operating as co-adjuvant for the care and management of skin lesions, and as co-adjuvant for the care and management of the biologic cicatrization process.

Some examples of such products are: Lyophilized Cicatrizer; Scaffold (surgical bio-scaffold); Disposable active patch.
- Lyophilized Cicatrizer: used as co-adjuvant for the care and management of skin lesions, such as those of contusion and trauma origin, those of systemic origin, such as ulcers bedsores, in addition to the management of burns and the losses of substances.
- Appearance: whitish lyophilized product shaped as crystallized powder.
- Packaging: sterile lyophilized product stored in disposable ampoules with easy open plug for an easy management of the product. The product can be dispensed directly from the ampoule on the lesion or through an adequately sized dispersing device.
- Scaffold (surgical bio-scaffold): used as co-adjuvant for the care and management of the biologic cicatrization process. The patch provides the necessary scaffold for recovering the loss of substance, operating as driver in the tissue repairing process, typical processes of precision medicine and of regenerative surgery.

The intrinsic properties of the snail drool optimized by the lyophilizing process contribute to accelerate the healing process of chronic skin lesions, such as ulcers and bedsores, of acute and traumatic injuries.

The patch can be used with success for managing both on the surface and deep burns.
- Appearance: patch, for example of a square shape with sizes of 5x5 cm, compact appearance without a prevailing side, suitable to various body seats, obtained from compacting the lyophilized product.
- Packaging: sterile patch stored in a transparent blister with easy-open back for an easy management of the product. The product can be laid directly on the lesion, to which a following advanced medication will occur according to a standard protocol.
- Disposable active patch: used as co-adjuvant for the care and management of skin lesions, above all those of contusion and trauma origin.
- Appearance: patch, for example of an hexagonal shape, with various sizes with perimeter adhesive.
- Packaging: disposable and single-package sterile patches stored in an adequately sized box. The product can be laid directly on the lesion.

Advantageously, the lyophilized snail drool according to the present invention allows obtaining, in a totally natural way, free from additives and/or manipulations, an innovative bio-material having concentrations which cannot be found in nature apart from synthesis products. The product can be, after suitable working, used in human and animal medicine as co-adjuvant of surface and deep, dermal and epidermal lesions, young or made chronical, of a physiologic or mechanical nature.

## Claims

1. Lyophilized snail drool to be used as cicatrizer and/or as regenerative surgical support, **characterized in that** it comprises the following percentages of components:
- Glycolic acid: included between 15% and 35%
- Allantoin: included between 2% and 8%
- Proteins: included between 2% and 6%
- Collagen: included between 0.5% and 1.5%
- Remaining: included between 50% and 80% of a matrix with very low molecular weight;
and **in that** it comprises:
- Glycolic acid: mean value 227464 mg/kg;
- Allantoin: mean value 46147 mg/kg;
- Total proteins: mean value 2.1 g/100g;
- Collagen: 1% m/m;
and has a pH of 3.5.

2. Lyophilized snail drool according to claim 1, **characterized in that** it comprises:
- Glycolic acid: about 25%
- Allantoin: about 5%
- Proteins: about 4%
- Collagen: 1%
- Remaining: 65% of a matrix with very low molecular weight.

3. Use of the lyophilized snail drool according to any one of the previous claims, as cicatrizing lyophilized product or as regenerative surgical support.

4. Regenerative surgical support obtained from compacting the lyophilized snail drool according to any one of claims 1 to 2, configured to be suited to various body seats and to provide the necessary scaffold for recovering losses of substances by operating as driver in a tissue repairing process.

5. Disposable active patch with adhesive configured to be laid directly on a lesion, comprising lyophilized snail drool according to any one of claims 1 to 2.

6. Process for producing lyophilized snail drool, **characterized in that** it comprises a step of lyophilizing snail drool extracted from snails.

7. Process for producing lyophilized snail drool according to claim 6, **characterized in that** the step of lyophilizing the extracted snail drool comprises:
- a step wherein the snail drool is stored in a refrigerating cell at a temperature included between 0 °C and -4 °C till freezing preparatory for lyophilizing;
- a step of freezing the snail drool at -35 °C;
- a step of lyophilizing, performed at a temperature of about -50° C and with a vacuum of about 0.020 mmbar.
